# EUROPEAN PATENT APPLICATION

(11) **EP 1 190 708 A1**
(43) Date of publication of application: **27.03.2002**
(21) Application number: 00120646.5
(22) Date of filing: 21.09.2000
(51) Int. Cl.: A61K 31/00, A61K 31/475, A61K 38/10, A61P 43/00, A61K 31/13

(54) **Medical use of substances**

(71) Applicant: Tinnitus Forschungs- und Entwicklungs GmbH, 80333 München (DE)
(72) Inventor: Ruppersberg, J. Peter, Prof.Dr., 72072 Tübingen (DE); Fakler, Bernd, 72076 Tübingen (DE)
(74) Representative: Patentanwälte Ruff, Wilhelm, Beier, Dauster & Partner

(57) **Abstract**

The invention relates to the use of substances for the manufacturing of a pharmaceutical composition or medicament for the treatment of disturbances or illnesses which are linked to malfunction of an ionotropic acetylcholine receptor and/or a calcium-activated potassium channel functionally associated with said acetylcholine receptor.

## Description

The invention relates to the use of substances for the manufacturing of a pharmaceutical composition or medicament for the treatment of disturbances or illnesses which are linked to malfunction of an ionotropic acetylcholine receptor. Furthermore, the invention relates to the use of substances for the manufacturing of a pharmaceutical composition or a medicament for the treatment of disturbances or illnesses which are linked to malfunction of a calcium-activated potassium channel functionally associated with an ionotropic acetylcholine receptor.

The acetylcholine receptor is a ligand-gate cation channel. It is a glycoprotein composed of different types of transmembraned polypeptides. Each of those polypeptides is coded by a separate gene, although they all show strong similarities in their amino acid sequences, implying that their genes have evolved from a single gene.

Like the voltage-gated Na⁺-channel, the acetylcholine-gated channel has a number of discrete alternative conformations and in the presence of the ligand jumps randomly from one to another, switching abruptly between closed and opened states. Once it has bound acetylcholine and made the transition to the opened stage, it remains open for a randomly variable length of time, averaging about one millisecond. Greatly prolongated exposure to acetylcholine causes the channel to enter a desensitized state. In the open conformation, the channel has a lumen at its extracellular end, narrowing towards cell interior to a small pore. The charge distribution in the channel wall is such that negative ions are excluded, while any positive ion can pass through. The normal traffic consists of Na⁺ and K⁺, together with some Ca²⁺. Since there is little selectivity among cations, their relative contributions to the current through the channel depends mainly on their concentrations and on the electrochemical driving forces. Opening of the acetylcholine receptor channel leads to a large net influx of positive ions, causing membran depolarization. This membran depolarization causes a signal transmission from one membran to another. A malfunction of this receptor can cause several diseases, and a malfunction of the acetycholine receptor in the brain is e.g. discussed as reason for Alzheimer disease.

It is the object of the invention to convert new scientific insights into a new strategy for the treatment of important diseases. This object is solved by the subject-matter of claims 1 and 10. Preferred embodiments are given in the dependent claims 2 to 9 and 11 to 17. The wording of all these claims is hereby incorporated into the content of the description by reference.

Surprisingly it was found, that the acetylcholine receptor in the inner ear comprises of two subunits, namely alpha9 and alpha10. These subunits can be blocked by memantine, an adamantane derivative known for treatment of tinnitus associated with a so-called positive recruitment and/or a reduction or failure of otoacoustic emissions (EP 0759 295).

Furthermore, it was found, that this acetycholine receptor in the inner ear is functionally associated with a calcium-activated potassium channel, namely of the SK subtype. These findings make it possible to use adamantane derivatives and other substances with the same blocking function for the treatment of disturbances that are linked to malfunction of this acetylcholine receptor and/or this calcium-activated potassium channel.

According to the invention, at least one substance is used for the manufacturing of a pharmaceutical composition or a medicament for the treatment of disturbances or illnesses which are linked to malfunction of at least one ionotropic acetylcholine receptor, wherein said substance is at least partly blocking at least one ionotropic acetylcholine receptor and wherein that disturbance or illness is not a tinnitus associated with a so-called positive recruitment and/or a reduction or failure of otoacoustic emissions. According to the present invention, the acetylcholine receptor preferably comprises at least one so-called alpha9-subunit and alternatively or in addition it comprises at least one so-called alpha10-subunit. The substance which is used for the manufacturing of a pharmaceutical composition or medicament can be used in the form of its pharmaceutical acceptable salts and/or optionally together with a pharmaceutically carrier.

According to the invention, the above mentioned acetylcholine receptor is preferably functionally associated with at least one calcium-activated potassium channel. This calcium-activated potassium channel can be characterized in that it is of SK (small conductance) subtype, wherein preferably that SK potassium channel is of the SK2 subtype.

According to the invention the substance used for the treatment of disturbances or illnesses linked to malfunction of at least one ionotropic acetylcholine receptor can be a strychnine, a peptide, preferably a polypeptide, or a polynucleotide encoding such peptide, preferably a polypeptide. That also can also be at least one adamantane derivative, especially those used in EP 0759 295. Furthermore, the substance can be a small molecular compound, preferably a small molecular compound with a molecular weight (MW) < 1000.

In other preferred embodiments according to the invention, the use of the substance is claimed for the manufacturing of a pharmaceutical composition or a medicament for the treatment of disturbances or illnesses linked to malfunction of at least one calcium-activated potassium channel functionally associated with a ionotropic acetylcholine receptor, wherein said substance is at least partly blocking at least one calcium-activated potassium channel functionally associated with an ionotropic acetylcholine receptor and wherein that disturbance or illness is not a tinnitus associated with a so-called positive recruitment and/or a reduction or failure of otoacoustic emissions.

According to the invention, this acetylcholine receptor which is functionally linked with a calcium-activated potassium channel preferably comprises at least one so-called alpha9-subunit and alternatively or in addition it comprises at least one so-called alpha10-subunit. The calcium-activated potassium channel is of the SK subtype, wherein preferably that SK potassium channel is of the SK2 subtype.

According to the invention the substance which at least partly blocks at least one calcium-activated potassium channel defined as above can be a peptide, preferably a polypeptide. This polypeptide can be apamine, which preferably blocks this channel, but also even up to now unknown compounds which block this channel are claimed for the inventive use. The substance can also be a polynucleotide encoding such a peptide, preferably polypeptide, or it can be a small molecular compound, preferably a small molecular compound with a molecular weight (MW) < 1000.

The invention can be useful for treatment of all kinds of disturbances or illnesses which are linked with malfunction of an ionotropic acetylcholine receptor as defined above or the calcium-activated potassium channel which is functionally associated with the above mentioned acetylcholine receptor and which is also defined above.

Finally, according to the invention it is possible to select the administration form of the substance. This form can be adapted to the age, sex or other characteristics of the patient, the severity of the disturbances or illnesses or other parameters. Conventional additives can be present. The dosage can be freely selected as a function of the clinical picture and the condition of the patient.

The described features and further features of the invention can be gathered from the following description of preferred embodiments in conjunction with the subclaims. The individual features can be implemented seperately or in the form of subcombinations.

### MATERIALS AND METHODS

### Electrophysiology on OHCs

Organs of Corti were dissected from the cochleae of Wistar rats (11-24 days old) as described previously (Oliver, D. and Fakler, B. 1999. J. Physiol. (Lond.) 519 pt. 3, 791-800; Oliver, D., Klöcker, N., Schluck, J., Baukrowitz, T., Rup-persberg, J.P. and Fakler, B. 2000. Neuron 26, 591-601). The electrophysiology on OHCs was also described previously (Oliver, D. et al., 2000, see above).

### Electrophysiology on heterologously expressed proteins

In vitro mRNA synthesis and oocyte injections were performed as previously described (Fakler, B., Brandle, U., Glowatzki, E., Weidemann, S., Zenner, H.P. and Ruppersberg, J.P. 1995. Cell 80, 149-154; Oliver, D. et al., 2000, see above). The electrophysiology on oocytes was also performed as described previously (Oliver, D. et al., 2000, see above).

### Figure legends:

Fig. 1: (A) Model for the inhibitory synaptic transmission at outer hair cells (OHCs) as derived from experiments with isolated cells. (B) Inhibitory postsynaptic currents (IPSCs) were inhibited by strychnine and apamine.

Fig. 2: Block of alpha9/alpha10 SK2 current responses to acetylcholine in Xenopus oocytes by strychnine.

Fig. 3: Memantine dose-responses determined for the inhibitory postsynaptic currents (IPSC) in outer hair cells.

Fig. 4: Inhibition of alpha9/alpha10 heteromeric acetylcholine receptors expressed in Xenopus oocytes by memantine.

### Experiment 1:

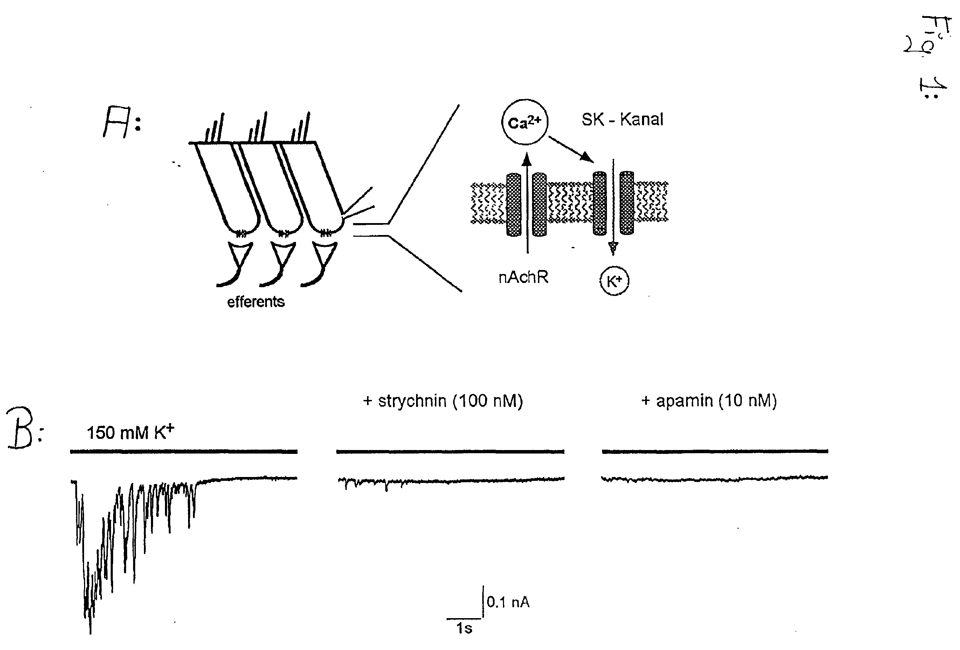

In Fig. 1A, a model is shown of how the inhibitory transmission at OHCs occurs. Ca²⁺ entering the postsynapse via the transmitter-activated nAChR opens SK channels and thus results in hyperpolarizing K⁺ currents (Art, J.J., Fettiplace, R. and Fuchs, P.A. 1984. J. Physiol. (Lond.) 356, 525-550; Yuhas, W.A. and Fuchs, P.A. 1999. J. Comp. Physiol. 18, 455-462).

In Fig 1B, postsynaptic currents were recorded from OHCs in voltage-clamp experiments when cells were held at -64 mV and the whole Corti preparation was superfused with high extracellular K⁺ (150 mM) to depolarize the presynapse. Application of high K⁺ and toxins as indicated by horizontal bars: current and time scaling as indicated.

Consistent with the model presented in Fig. 1A, the postsynaptic currents were inhibited by application of either the acetylcholine blocker strychnine (Elgoyhen, A.B., Johnson, D.S., Boutler, J., Vetter, D.E. and Heinemann, S. 1994. Cell 79, 705-715) or the SK channel blocker apamine (complete inhibition at 100 nM and 10 nM, respectively; Fig. 1B).

### Experiment 2:

For further characterization of strychnine treatment on the nAChR, the mRNA of alpha9/alpha10 subunits of the acetylcholine receptor were injected in oocytes of Xexopus laevis.

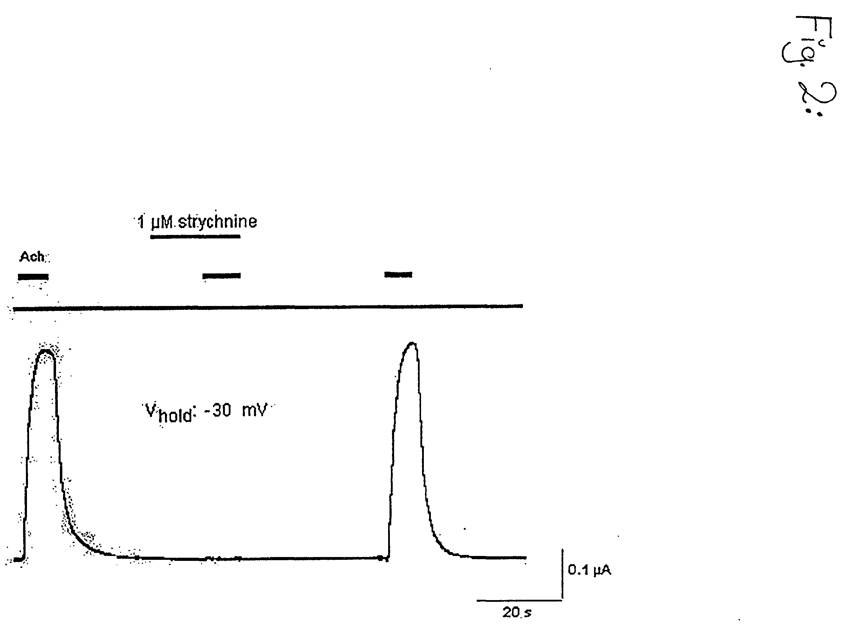

After expression of these subunits, they were investigated by treatment with strychnine in voltage-clamp experiment. The cells were held at -30 mV. Current and time scaling are as indicated. As is shown in Fig. 2, similar results were obtained as in Fig. 1. Consistent with the model shown above, the current responses at the acetylcholine receptor could be blocked reversibly by application with the acetylcholine blocker strychnine. A complete inhibition occurs at 1 µM, and after washout of strychnine for 3 min. the receptor was again functionally active, indicating a reversible block of the receptor.

### Experiment 3:

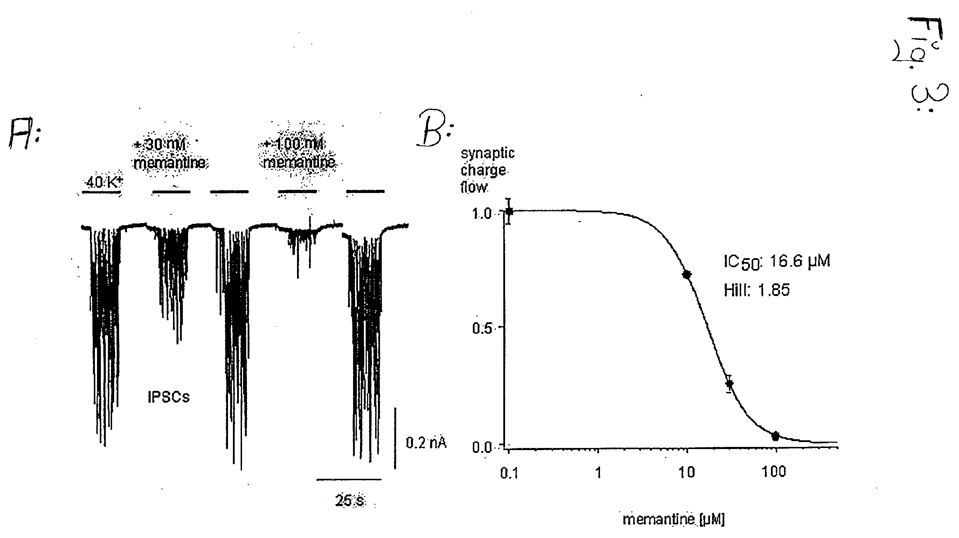

In Fig 3, postsynaptic currents were recorded from outer hair cells (OHCs) in voltage-clamp experiments when cells were held at -64 mV and the whole Corti preparation was superfused with high extracellular K⁺ to depolarize the presynapse. Application of high K⁺ and toxins as indicated by horizontal bars: current and time scaling as indicated. The postsynaptic currents were reversibly inhibited by application of memantine, whereas there is a dose-dependent response of the OHCs (30 mM and 100 mM, respectively). This dose-dependent response determined for the IPSC in OHCs is illustrated in Fig. 3B. There it is shown, that in OHCs memantine has an IC₅₀ of approximately 16.6 µM.

### Experiment 4:

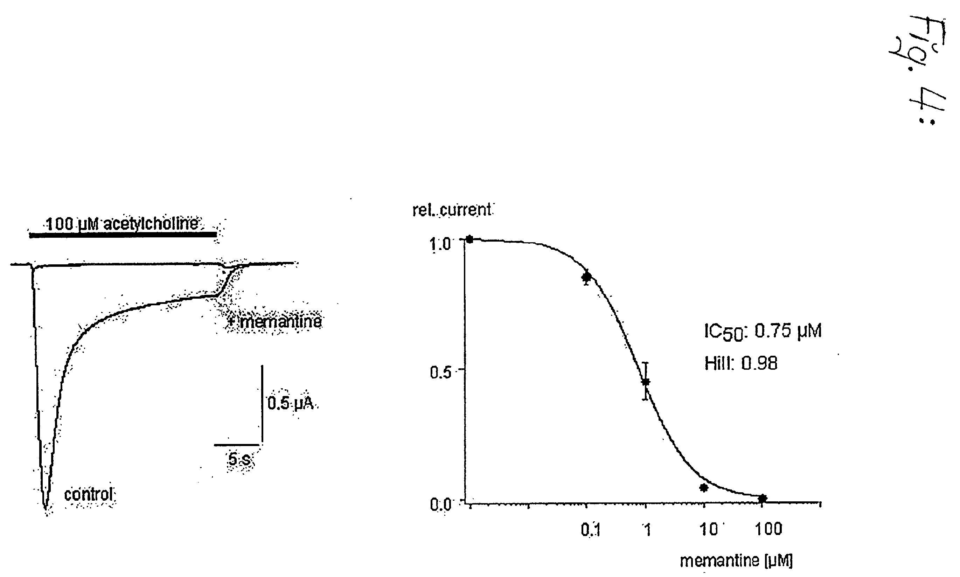

For further characterization of memantine treatment on the neuronal acetylcholine receptor (nAChR), the mRNA of alpha9-/alpha10 subunits of the acetylcholine receptor were injected in oocytes of Xexopus laevis. After expression of that subunits, these subunits were investigated by treatment with memantine in voltage-clamp experiment. The cells were held at -30 mV. Current and time scaling are as indicated. As is shown in Fig. 4, similar results were obtained as in Fig. 3. In this experiment the polarization potential of the cells was meassured after application of 100 µM acetylcholine in the presence or absence of memantine. Consistent with the results obtained in Fig. 3, the current responses at the acetylcholine receptor could be blocked by application with memantine. A halfmaximal inhibition occurs at concentrations of approximately 1 µM, as is illustrated in Figur 4B.

## Claims

1. Use of a substance for the manufacturing of a pharmaceutical composition or medicament for the treatment of disturbances or illnesses linked to malfunction of at least one ionotropic acetylcholine receptor, wherein said substance is at least partly blocking said acetylcholine receptor and wherein said disturbance or illness is not a tinnitus associated with a so-called positive recruitment and/or a reduction or failure of otoacoustic emissions.

2. Use according to claim 1, **characterized in that** said acetylcholine receptor comprises at least one alpha9-subunit.

3. Use according to claim 1 or claim 2, **characterized in that** said acetylcholine receptor comprises at least one alpha10-subunit.

4. Use according to one of the preceding claims, **characterized in that** said acetylcholine receptor is functionally associated with at least one calcium-activated potassium channel.

5. Use according to claim 4, **characterized in that** said calcium-activated potassium channel is of SK subtype, wherein preferably said SK potassium channel is SK2.

6. Use according to one of the preceding claims, **characterized in that** said substance is a strychnine derivative.

7. Use according to one of claims 1 to 5, **characterized in that** said substance is a peptide, preferably a polypeptide.

8. Use according to one of claims 1 to 5, **characterized in that** said substance is a polynucleotide encoding a peptide, preferably a polypeptide, of claim 7.

9. Use according to one of claims 1 to 5, **characterized in that** said substance is a small molecular compound, preferably a small molecular compound with a molecular weight (MW) < 1000.

10. Use of a substance for the manufacturing of a pharmaceutical composition or medicament for the treatment of disturbances or illnesses linked to malfunction of at least one calcium-activated potassium channel functionally associated with an ionotropic acetylcholine receptor, wherein said substance is at least partly blocking said potassium channel and wherein said disturbance or illness is not a tinnitus associated with a so-called positive recruitment and/or a reduction or failure of otoacoustic emissions.

11. Use according to claim 10, **characterized in that** said acetylcholine receptor comprises at least one alpha9-subunit.

12. Use according to claim 10 or claim 11, **characterized in that** said acetylcholine receptor comprises at least one alpha10-subunit.

13. Use according to one of claims 10 to 12, **characterized in that** said calcium-activated potassium channel is of SK subtype, wherein preferably said SK potassium channel is SK2.

14. Use according to one of claims 10 to 13, **characterized in that** said substance is a peptide, preferably a polypeptide.

15. Use according to claim 14, **characterized in that** said peptide is apamine.

16. Use according to one of claims 10 to 13, **characterized in that** said substance is a polynucleotide encoding a peptide, preferably a polypeptide, of claim 14.

17. Use according to one of claims 10 to 13, **characterized in that** said substance is a small molecular compound, preferably a small molecular compound with a molecular weight (MW) < 1000.
